# EUROPÄISCHE PATENTSCHRIFT

(11) **EP 0 302 287 B1**
(45) Veröffentlichungstag und Bekanntmachung des Hinweises auf die Patenterteilung: **22.12.1993**
(21) Anmeldenummer: 88111473.0
(22) Anmeldetag: 16.07.1988
(51) Int. Cl.: G01N 33/52

(54) **Testträger zur Bestimmung eines Analyten aus Blut und Verfahren zu seiner Herstellung**
Test element for determining an analyte in blood and method for its preparation
Diagnostique pour la détermination d'analyte dans le sang et procédé pour sa préparation

(30) Priorität: 04.08.1987 DE 3725766
(43) Veröffentlichungstag der Anmeldung: 08.02.1989
(73) Patentinhaber: BOEHRINGER MANNHEIM GMBH, 68298 Mannheim (DE)
(72) Erfinder: Knappe, Wolfgang-Reinhold, Dr. rer. nat., D 6842 Bürstadt 1 (DE); Scheithauer, Dieter, D-6832 Hockenheim (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 016 387
- EP-A- 0 226 767
- EP-A- 0 271 854
- WO-A-84/02192
- DE-A- 3 237 233
- PATENT ABSTRACTS OF JAPAN Band 11, Nr. 334 (P-631)(2781), 31. Oktober 1987; && JP-A-62116258 (FUJI PHOTO FILM CO.) 27.05.1987

## Beschreibung

Die Erfindung betrifft einen Testträger zur Bestimmung eines Analyten aus Vollblut mit Hilfe von in dem Testträger enthaltenen Reagenzien, mit einer Erythrozytenabtrenneinrichtung, die eine Blutaufgabeseite und eine Auswerteseite aufweist, wobei auf der Blutaufgabeseite Vollblut zugeführt wird und die Auswerteseite einer Nachweiseinrichtung zugewandt ist, in der infolge der Reaktion der Reagenzien mit dem Analyten eine optisch nachweisbare Veränderung stattfindet. Weiter richtet sich die Erfindung auf ein Verfahren zum Herstellen eines derartigen Testträgers.

Zur qualitativen oder quantitativen analytischen Bestimmung von Bestandteilen von Blut werden in jüngerer Zeit zunehmend sogenannte trägergebundene Tests verwendet. Bei diesen sind Reagenzien in entsprechenden Schichten eines festen Testträgers eingebettet, der mit der Probe in Kontakt gebracht wird. Die Reaktion von Probe und Reagenzien führt zu einer optisch nachweisbaren Veränderung, insbesondere einem Farbumschlag, welcher visuell oder mit Hilfe eines Gerätes, meistens reflexionsphotometrisch, ausgewertet werden kann. Statt zu einem Farbumschlag kann die Reaktion auch zum Entstehen oder zur Veränderung eines anderen optisch nachweisbaren Signals führen, beispielsweise einer Fluoreszenz oder einer Lumineszenz. Testträger sind häufig als Teststreifen ausgebildet, die im wesentlichen aus einer länglichen Tragschicht aus Kunststoffmaterial und darauf angebrachten Testfeldern bestehen. Es sind jedoch auch Testträger bekannt, die als quadratische oder rechteckige Plättchen ausgebildet sind. Trägergebundene Tests zeichnen sich insbesondere durch die Einfachheit der Handhabung aus. Umso bedauerlicher ist es, daß bei den meisten bisher bekannten trägergebundenen Tests das Blut nicht unmittelbar als sogenanntes Vollblut verwendet werden kann. Es ist vielmehr erforderlich, die roten Blutkörperchen (Erythrozyten) abzutrennen, um farbloses Plasma bzw. Serum zu gewinnen. Dies geschieht üblicherweise durch Zentrifugieren, d.h. es ist ein zusätzlicher Handhabungsschritt erforderlich. Außerdem steht ein Gerät zum Zentrifugieren nicht überall zur Verfügung, zumal trägergebundene Tests in zunehmendem Maße auch für Laien angeboten werden. Die Zentrifugation erfordert eine relativ große Probenmenge, während andererseits das Streben in der klinischen Diagnostik dahingeht, mit einem kleinen Blutstropfen auszukommen, wie er durch einen Stich in den Finger gewonnen werden kann.

Es hat daher nicht an Versuchen gefehlt, Testträger zur Verfügung zu stellen, welche analytische Bestimmungen unmittelbar aus Blut ermöglichen.

So ist aus der DE-B-1 598 153 ein Testträger mit einer aus einer wässrigen Dispersion natürlicher oder synthetischer Polymere hergestellten Filmschicht bekannt, in der sich die für den Nachweis notwendigen Reagenzien befinden. Mit diesem Testträger können bestimmte analytische Bestimmungen, insbesondere die Bestimmung von Glucose, unmittelbar aus Blut vorgenommen werden. Bei anderen analytischen Bestimmungen werden mit einem derartigen Testträger jedoch keine guten Resultate erhalten, was darauf zurückzuführen sein dürfte, daß die Inhaltsstoffe der Probe nicht in ausreichendem Maße in diesen Film eindringen können.

Aus der EP-A-16 387 ist eine ebenfalls auf der Verwendung eines Dispersionsfilmbildners basierende Testträgerschicht bekannt, welche dieses Problem dadurch vermeidet, daß sie relativ große Mengen an (insbesondere anorganischen) kleinen Partikeln enthält. Damit wird zwar eine universell einsetzbare Testschicht erhalten, die jedoch keine Bestimmung aus Vollblut erlaubt, weil in diese Schicht nicht nur größere zu analysierende Probenbestandteile, sondern auch Erythrozyten ungehindert eindringen.

Aus der EP-A 45 476 ist es bekannt, Glasfasern zur Gewinnung von Serum oder Plasma auf einem Testträger einzusetzen. Diese Lösung ist zwar universell einsetzbar, jedoch ist es erforderlich, die Glasfaserschichten in geeigneter Weise auf dem Testträger unterzubringen. Dadurch entsteht ein verhältnismäßig komplizierter Testträgeraufbau, und das Verfahren der Herstellung ist aufwendig.

Auch soweit bisher Testträger der eingangs bezeichneten Art vorgeschlagen wurden, bei denen auf die eine Seite Blut ohne vorherige Erythrozytenabtrennung aufgegeben werden kann und auf der anderen Seite die Auswertung erfolgt, wobei sich zwischen der Blutaufgabeseite und der Auswerteseite eine Erythrozytenabtrenneinrichtung befindet, konnten diese Versuche bisher nicht befriedigen.

In der US-A-36 63 374 und in der US-A-42 56 693 wird ein Membranfilter eingesetzt, um den Durchtritt der Erythrozyten von der Blutaufgabeseite auf die Auswerteseite zu verhindern. Zwar sind Membranfilter für das Abfiltern von Erythrozyten grundsätzlich geeignet. Ihre Verwendung in Testträgern hat sich jedoch nicht bewährt. Das gleiche gilt für die ebenfalls in diesen US-Patenten erwähnte Kombination des Membranfilters mit einer vorgelagerten Glasfaserschicht, welche das Verstopfen des Membranfilters mit gröberen Teilchen verhindern soll. Die Herstellung derartiger Testträger wäre sehr aufwendig, ohne daß eine befriedigende Funktion erreicht wird.

In der US-A-4 069 817 und mehreren anderen US-Patenten der gleichen Anmelderin wird ebenfalls die Möglichkeit angesprochen, in einem Testträger eine den Durchtritt von Erythrozyten verhindernde Zwischenschicht vorzusehen, welche zugleich strahlenblockierende Bestandteile enthält, um sicherzustellen, daß die Lichtstrahlen des Auswertegerätes nicht in die erythrozytenhaltige Schicht vordringen können. In dieser Schrift sind jedoch keinerlei Angaben enthalten, wie die Filterung der Erythrozyten erreicht werden könnte.

Aufgabe der vorliegenden Erfindung ist es daher, einen Testträger zur Verfügung zu stellen, mit dem die Durchführung medizinisch-diagnostischer Bestimmungen unmittelbar aus Vollblut möglich ist, wobei der Testträger einfach zu handhaben und einfach und kostengünstig herzustellen sein soll.

Die Aufgabe wird bei einem Testträger der eingangs bezeichneten Art dadurch gelöst, daß die Erythrozytenabtrenneinrichtung einen Schichtverbund einschließt, der eine erste Zone, welche einen polymeren Filmbildner, Kieselgur und ein Pigment enthält und eine darauf unter Bildung eines Übergangsbereichs durch Flüssigbeschichten geformte, einen polymeren Filmbildner enthaltende zweite Zone aufweist, wobei die erste Zone der Blutaufgabeseite und die zweite Zone der Auswerteseite der Erythrozytenabtrenneinrichtung zugewandt ist.

Durch die Erfindung wird ein Testträger zur Vollblutanalyse zur Verfügung gestellt, dessen Erythrozytenabtrenneinrichtung sich mit einer einfach durchzuführenden Doppelbeschichtung kostengünstig herstellen läßt. Der Durchtritt des Plasmas von der ersten Zone in die Auswertezone erfolgt in wenigen Sekunden, so daß eine schnelle Auswertung möglich ist. Die Handhabung ist einfach, insbesondere muß das aufgebrachte Blut nicht abgewischt oder abgewaschen werden, wie dies bei vorbekannten Teststreifen häufig erforderlich war.

Das erfindungsgemäße Verfahren zur Herstellung eines solchen Testträgers erfordert zwei verschiedene Beschichtungsmassen, welche in getrennten Beschichtungsschritten jeweils zu einer dünnen Schicht ausgeformt werden. Die erste Beschichtungsmasse enthält, neben einem in der Trägerflüssigkeit dispergierten oder gelösten polymeren Filmbildner, Kieselgur, ein Pigment und bekannte Hilfsstoffe, wie beispielsweise Puffer, Netzmittel, Verdickungsmittel, Entschäumer und dergleichen. Die zweite Beschichtungsflüssigkeit enthält ebenfalls einen dispergierten oder gelösten polymeren Filmbildner.

Zur Herstellung eines aus einer ersten Zone und einer zweiten Zone bestehenden Schichtverbundes wird zunächst die erste Beschichtungsmasse auf einer Unterlage in einer dünnen Schicht ausgeformt und getrocknet. Danach wird auf dieser Schicht die zweite Beschichtungsmasse zu einer dünnen Schicht ausgeformt und getrocknet. Dabei dringen Bestandteile der zweiten Beschichtungsflüssigkeit in die zuerst ausgeformte Schicht ein. Die erste Zone und die zweite Zone sind deswegen nicht durch eine scharfe Grenze voneinander getrennt, sondern es bildet sich ein Übergangsbereich, in dem sie fließend ineinander übergehen. Es ist vorteilhaft, bei der Aufbringung der Schichten bestimmte Viskositätsbereiche der beiden Beschichtungsmassen einzuhalten. Die erste Beschichtungsmasse sollte beim Aufbringen auf die Unterlage eine Viskosität von 300-3000 mpas (Millipascalsekunden) bei einem Schergefälle von 492 s⁻¹ haben. Für die zweite Beschichtungsmasse sollte der entsprechende Wert bei 100-1000 mpas liegen. Beide Viskositäten sind nach der Deutschen Industrie Norm (DIN) 53019 zu bestimmen.

Geeignete polymere Filmbildner sind bevorzugt organische Kunststoffe, wie Polyvinylester, Polyvinylacetate, Polyacrylester, Polymethylacrylsäure, Polycrylamide, Polyamide, Polystyrol. Außer Homopolymeren sind besonders auch Mischpolymerisate, z.B. von Butadien und Styrol oder von Maleinsäureester und Vinylacetat sowie Terpolymere geeignet. Es können jedoch auch weitere filmbildende, natürliche oder synthetische organische Polymere sowie Mischungen derselben verwendet werden. Gelatine ist nicht geeignet.

Die Filmbildner können in geeigneten organischen Lösungsmitteln gelöst sein. Oft ist es vorteilhaft, eine Dispersion eines geeigneten Filmbildners zu verwenden. Die bevorzugte Trägerflüssigkeit ist in diesem Falle Wasser.

Dispersionsfilmbildner enthalten submikroskopische, in der Trägerflüssigkeit unlösliche Polymerteilchen, welche in feinster Verteilung in der Trägerflüssigkeit dispergiert sind. Wird bei der Filmbildung die Flüssigkeit durch Verdampfen bzw. Verdunsten entfernt, so nähern sich die Teilchen und berühren sich schließlich. Durch die dabei auftretenden großen Kräfte und einen mit der Filmbildung einhergehenden Gewinn an Oberflächenenergie wachsen die Teilchen zu einer weitgehend geschlossenen Filmschicht zusammen. Nähere Einzelheiten hierzu sind beispielsweise dem Artikel "Latex Film Formation" von J.W. Vanderhoff in Polymer News 1977, Seite 194 - 203 zu entnehmen.

Kieselgur wird auch als Diatomeenerde bezeichnet. Es handelt sich um aus den Kieselsäuregerüsten der Diatomeenarten entstandene Ablagerungen, die an verschiedenen Orten abgebaut werden. Die bevorzugt eingesetzte Kieselgur hat einen mittleren Teilchendurchmesser von 5 - 15 µm, wobei diese Werte mit einem Laser-Granulometer Typ 715 bestimmt wurden, welches von der Firma Pabisch, München, BRD vertrieben wird.

Das Pigment besteht bevorzugt aus Teilchen mit einem mittleren Durchmesser zwischen etwa 0,2 und 0,7 µm. Besonders geeignet ist beispielsweise Titandioxid. Es sind jedoch auch andere Pigmente geeignet, wobei deren Teilchengrößen üblicherweise näherungsweise in dem angegebenen Bereich liegen, welcher dem Wellenlängenspektrum des sichtbaren Lichtes etwa entspricht. Dadurch wird eine maximale Lichtstreuung und damit ein hochdeckendes Pigment erreicht.

Die Reaktionszeit in dem Schichtverbund wird dadurch verkürzt, daß dieser vorzugsweise maximal 0,6 mm, besonders bevorzugt nur maximal 0,2 mm dick ist.

Als Unterlage zum Ausformen der ersten Beschichtungsmasse kann beispielsweise eine Platte aus Glas oder aus einem anderen Material gewählt werden, von dem sich die Filmschicht leicht ablösen läßt. Dadurch ist es möglich, den fertiggestellten Schichtverbund abzunehmen und beispielsweise auf einer durchsichtigen Trägerfolie zu montieren, wobei die Auswerteseite der Trägerfolie zugewandt ist, so daß die Blutaufgabeseite des Schichtverbunds frei zugänglich ist.

Wesentlich einfacher in der Herstellung und deswegen bevorzugt ist es aber, den Schichtverbund auf einer porösen Tragschicht zu befestigen,wobei die Blutaufgabeseite der Tragschicht zugewandt ist. Dies läßt sich bevorzugt dadurch bewerkstelligen, daß man als Unterlage für das Ausformen der ersten Beschichtungsmasse unmittelbar die poröse Tragschicht verwendet. Bei einem derartigen Schichtverbund ist ein freier Luftzutritt zu der Nachweiszone möglich, wodurch die Reaktionszeit in dieser Zone in vielen Fällen erheblich verkürzt wird. Dadurch sind Endpunktbestimmungen möglich.

Als poröse Tragschicht eignet sich grundsätzlich jede offene, flächige Verbundstruktur, d.h. jede Struktur, die flächig ausgedehnt und ausreichend offen ist, so daß sie von Blut ausreichend schnell durchdrungen wird. Geeignet wäre beispielsweise eine siebartige Struktur aus einem Kunststoffmaterial mit sehr vielen dicht beieinander angeordneten Löchern. Bevorzugt besteht der poröse Träger jedoch aus einem textilen Material, insbesondere einem Gewebe oder Gewirke, welches beispielsweise aus Polyamid, Polyester oder Seide hergestellt sein kann. Evtl. kann auch ein Vlies oder Papier verwendet werden. Geeignete Materialien sind in der EP-A-113 896 beschrieben.

Die Erfindung wird im folgenden anhand eines in den Figuren schematisch dargestellten Ausführungsbeispiels näher erläutert; es zeigen:
- Fig. 1: einen erfindungsgemäßen Testträger in perspektivischer Darstellung,
- Fig. 2: einen Schnitt durch einen Testträger nach Fig. 1 entlang der Linie S/S,
- Fig. 3: einen Schnitt entsprechend Fig. 2 mit einem alternativen Schichtaufbau
- Fig. 4: eine perspektivische Darstellung einer alternativen Ausführungsform eines erfindungsgemäßen Testträgers.

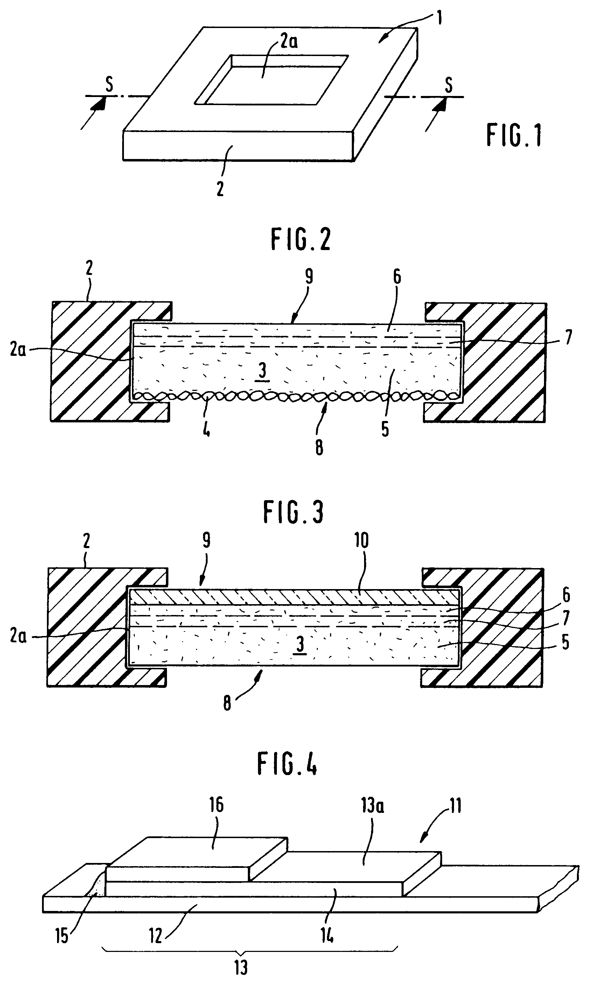

Der in Fig. 1 dargestellte Testträger 1 besteht im wesentlichen aus einem beispielsweise aus Kunststoff hergestellten Rahmen 2 und einem in diesem Rahmen gefaßten mehrschichtigen Testfeld 2a.

Das mehrschichtige Testfeld 2a besteht im dargestellten Fall im Querschnitt aus einem als Erythrozytenabtrenneinrichtung wirkenden Schichtverbund und einer porösen Tragschicht 4. Der Schichtverbund 3 umfaßt eine erste Zone 5 und eine zweite Zone 6, sowie einen durch eine gestrichelte Doppellinie angedeuteten Übergangsbereich 7 zwischen beiden Zonen. Die poröse Tragschicht 4 ist der Blutaufgabeseite 8 des Schichtverbundes 3 zugewandt, während die zweite Zone 6 der Auswerteseite 9 zugewandt ist.

Selbstverständlich kann das mehrschichtige Testfeld 2a weitere Schichten enthalten. Das dargestellte Beispiel ist insofern besonders einfach aufgebaut, als die erste Zone 5 und die zweite Zone 6 zugleich die Reagenzien für die Nachweisreaktion enthalten. Es kann jedoch auch zweckmäßig sein, die Funktionen "Erythrozytenabtrennung" und "Nachweisreaktion" zu trennen und für letztere zusätzliche Schichten vor allem auf der Nachweisseite, evtl. aber auch auf der Blutaufgabeseite vorzusehen.

Zur Durchführung einer analytischen Bestimmung wird eine gegebenenfalls vordosierte Blutmenge auf die Blutaufgabeseite 8 aufgebracht, wobei der Testträger üblicherweise mit dieser Seite nach oben gehalten wird. Das Blut dringt durch die poröse Tragschicht 4 in die Basiszone 5 ein. Aufgrund des erfindungsgemäßen Aufbaus des Schichtverbundes bleiben die Erythrozyten beim weiteren Eindringen zurück, so daß kein roter Blutfarbstoff in die Nachweiszone 6 gelangt. Die optische Auswertung erfolgt von der Auswerteseite 9 her. Das Pigment blockiert dabei die Strahlung soweit, daß sie nicht in Bereiche des Schichtverbundes vordringen kann, in denen sich roter Blutfarbstoff befindet. Die Analyse wird folglich nicht durch diesen verfälscht.

Der erfindungsgemäße Schichtverbund erreicht in seiner Gesamtheit also das, was beispielsweise in der eingangs erwähnten US-A-4 069 817 gefordert wird, ohne daß geeignete Mittel angegeben werden, nämlich die Erythrozyten aus der eindringenden Blutprobe abzufiltern und zugleich die zur optischen Auswertung notwendige Lichtstrahlung so zu blockieren, daß sie nicht in den Bereich des Schichtverbundes eindringt, in dem sich roter Blutfarbstoff befindet.

Wodurch die Filterwirkung im einzelnen erreicht wird, ist nicht vollständig aufgeklärt. Zum einen läßt sich feststellen, daß eine die Zusammensetzung der ersten Zone 5 aufweisende (beispielsweise auf eine poröse Tragschicht aufgebrachte) Schicht keine ausreichende Erythrozyten-Abtrenneigenschaft hat. Andererseits läßt sich feststellen, daß ein erheblicher Teil der zweiten Beschichtungsmasse beim Ausformen auf der darunterliegenden Schicht in diese eindringt. Es ist also zu vermuten, daß die Teilchen des dispergierten oder gelösten Polymers aus der zweiten Beschichtungsmasse in die zuvor hergestellte Schicht eindringen, wobei sich ein Gradient des Polymers in dem Übergangsbereich 7 zwischen der zweiten Zone 6 und der ersten Zone 5 einstellt. Es ist anzunehmen, daß dadurch die verhältnismäßig offene Struktur der ersten Zone 5 gerade soweit geschlossen wird, daß einerseits die Erythrozyten zurückgehalten werden, daß aber andererseits auch verhältnismäßig große zu analysierende Bestandteile der Probe bis in die zweite Zone 6 vordringen können.

Einzelheiten des chemischen Ablaufs der analytischen Bestimmung sind für die Erfindung nicht wesentlich. Wesentlich ist insoweit lediglich, daß die für die gesuchte Konzentration charakteristische optisch nachweisbare Veränderung in der zweiten Zone 6 oder in einer weiteren auf der gleichen Seite des Übergangsbereichs 7 angeordneten Schicht des Testträgers und nicht in der ersten Zone 5 stattfindet. Bei zahlreichen in der klinischen Chemie üblichen Testverfahren wird beispielsweise als abschließende Verfahrensstufe eine farbbildende Substanz (beispielsweise ein chromogenes Substrat eines an der Reaktion beteiligten Enzyms) gebildet oder so umgesetzt, daß es seine Farbe ändert. Eine solche Reaktionskomponente kann man als "ein optisch nachweisbares Signal produzierende Komponente", kurz auch signalproduzierende Komponente (SPK) bezeichnen.

Bevorzugt befindet sich eine solche Komponente in der zweiten Zone 6 des Schichtverbundes 3. Vorzugsweise ist sie in der zweiten Beschichtungsmasse enthalten. Es ist jedoch auch möglich, die zweite Zone 6 nachträglich mit einer SPK zu imprägnieren oder zu besprühen. Dies ist insbesondere dann zweckmäßig, wenn die zweite Beschichtungsmasse auf Basis eines organisch gelösten Filmbildners hergestellt ist.

Die SPK muß jedoch nicht notwendigerweise in der zweiten Zone 6 von Anfang an vorhanden sein. Vielmehr sind Testführungen bekannt, bei denen solche Komponenten in einer anderen Schicht eines mehrschichtigen Testträgers gebildet werden oder vorhanden sind und im Zuge des Reaktionsablaufs in den eigentlichen Nachweisbereich gelangen. Auch auf derartige Verfahren ist die Erfindung anwendbar, wobei die Komponenten anfangs in der ersten Zone 5, der porösen Tragschicht 4 oder einer weiteren vorgelagerten Schicht enthalten sein können.

Im einzelnen wird der erfindungsgemäße Schichtverbund zweckmäßigerweise in der Form hergestellt, daß die erste Beschichtungsmasse zunächst auf eine langsam transportierte Bahn eines als poröse Tragschicht 4 geeigneten Materials über dessen volle Breite aufgetragen wird. Dabei hat die Beschichtungsmasse eine etwa honigartige Viskosität, so daß sie überwiegend auf einer Seite des textilen Tragschichtmaterials bleibt, jedoch in die Zwischenräume zwischen dessen bevorzugt multifilen Fäden einsinkt. Beim fertigen Produkt ist von der Blutaufgabeseite her das Material der ersten Zone in den Zwischenräumen der textilen Struktur zu erkennen, jedoch sollte es nicht dessen Fäden insgesamt umhüllen.

Die Verbindung zwischen der Tragschicht und der die erste Zone bildenden Beschichtung ist so fest, daß sie ohne Zerstörung nicht getrennt werden kann.

Je größer der Anteil an Pigment in der ersten Beschichtungsmasse ist, desto besser werden die Erythrozyten zurückgehalten, desto langsamer dringt aber auch das Plasma bis in die Auswertezone vor. Bevorzugt stehen die Kieselgur und das Pigment in der ersten Beschichtungsmasse in einem Gewichtsverhältnis von 1:0,5 bis 1:2, besonders bevorzugt in einem Gewichtsverhältnis von 1:0,8 bis 1:1. Ein entsprechendes Gewichtsverhältnis stellt sich dann selbstverständlich auch in der ersten Zone 5 ein. In diesem Zusammenhang ist anzumerken, daß alle Konzentrationsangaben der Zonen 5 und 6 sich auf deren außerhalb des Übergangsbereiches 7 liegende Teile beziehen.

Die Kieselgur und der polymere Filmbildner in der ersten Beschichtungsmasse und demzufolge auch in der ersten Zone 5 stehen bevorzugt in einem Gewichtsverhältnis von 1:0,2 bis 1:0,9 zueinander.

Nach dem Aufbringen der ersten Beschichtungsmasse wird diese zu einer dünnen Schicht ausgeformt, wobei zweckmäßigerweise eine sogenannte Rakel, d.h. ein über der transportierten Bahn der porösen Tragschicht angeordnetes Abstreiflineal verwendet wird, um die gewünschte Schichthöhe einzustellen.

Entsprechend wird in einem zweiten zweckmäßigerweise wegen der notwendigen verhältnismäßig langen Trocknungszeiten von dem ersten völlig getrennten Arbeitsschritt auf den Verbund von poröser Tragschicht und der darauf angeordneten, aus der ersten Beschichtungsmasse gebildeten, Schicht die zweite Beschichtungsmasse in entsprechender Weise aufgetragen. Diese zweite Schicht sollte sehr dünn aufgetragen werden. Je mehr Polymer aufgetragen wird, desto stärker werden die Erythrozyten zurückgehalten. Zugleich ist aber eine langsamere Bildung des optischen Signals zu beobachten. Bevorzugt wird der polymere Filmbildner in der zweiten Beschichtungsmasse mit einem Flächengewicht von maximal 200g/m², vorzugsweise maximal 100g/m² aufgetragen.

Wie erwähnt, hat sich in der Praxis gezeigt, daß die zweite Beschichtungsmasse in erheblichem Umfang in die darunterliegende Schicht eindringt. So wurde beispielsweise bei einer eingestellten Höhe des Beschichtungsspalts von 10 µm ein Verbrauch an zweiter Beschichtungsmasse gefunden, welcher einer 50 µm hohen Schicht entspricht.

Wie oben erwähnt kann der Schichtverbund 3 auch beispielsweise auf einer Glasplatte hergestellt werden, von der er sich nach der Herstellung leicht abnehmen läßt. Da er jedoch mechanisch nicht stabil ist, ist es zweckmäßig, ihn auf ein Trägermaterial aufzubringen. Fig. 3 zeigt eine alternative Ausführungsform eines erfindungsgemäßen Testträgers bei der als Trägermaterial eine durchsichtige Folie 10 zur Anwendung kommt, welche mit der zweiten Zone 6 verklebt ist. Eine derartige Ausführungsform kann in Fällen zweckmäßig sein, in denen die poröse Tragschicht 4 stören würde.

Der erfindungsgemäße Schichtverbund kann in Testträgern von sehr verschiedenem äußerem Aufbau eingesetzt werden.

Wesentlich ist lediglich, daß das Blut der ersten Zone 5 zugeführt wird und daß die Auswertung auf der Seite der zweiten Zone 6 erfolgt. Im übrigen können jedoch zahlreiche weitere konstruktive Merkmale, Schichten oder Reagenzien verwendet werden.

Fig. 4 zeigt beispielsweise einen ähnlich einem konventionellen Teststreifen geformten Testträger 11 mit einer schmalen, langgestreckten Basisfolie 12, die der Handhabung dient.

Auf der Basisfolie 12 ist ein Testbezirk 13 zu erkennen, in welchem eine Flüssigkeitstransportschicht 14 beispielsweise mit einem Schmelzkleberstreifen 15 auf die Basisfolie 12 aufgeklebt ist. Die Flüssigkeitstransportschicht 14 wird teilweise von dem Testfeld 16 überdeckt, welches im Querschnitt entsprechend dem Testfeld 2a in Fig. 2 aufgebaut ist. Es ist mit der porösen Tragschicht 4 nach unten ebenfalls mittels des Schmelzkleberstreifens 15 auf der Basisfolie 12 so befestigt, daß er in Flüssigkeitskontakt zu der Flüssigkeitstransportschicht 14 steht.

Bei dem in Fig. 4 dargestellten Testträger wird die Blutprobe auf den nicht von dem Schichtverbund 16 überlappten Teilbereich 13a der Flüssigkeitstransportschicht aufgegeben und dringt von dort in der Flüssigkeitstransportschicht (unter der Wirkung der Kapilarkraft) in den Bereich unter dem Schichtverbund 16, so daß das Blut in die erste Zone dieses Schichtverbundes 16 eindringen kann.

Der in Fig. 4 dargestellte Aufbau hat den Vorteil, daß die Blutaufgabe und die Auswertung von der gleichen Testträgerseite her erfolgen.

Die Erfindung kann besonders vorteilhaft im Zusammenhang mit dem in der deutschen Patentanmeldung P 36 43 516 beschriebenen Testträgeraufbau verwendet werden. Auf diese Patentanmeldung wird vollinhaltlich Bezug genommen.

Die folgenden Beispiele dienen der weiteren Erläuterung der Erfindung.

### Beispiel 1

### Herstellung eines Testfelds für einen Testträger zum Glucosenachweis aus Blut

198 g Acrylsäureester-Copolymer-Dispersion (Acronal 14D der BASF, Ludwigshafen, Bundesrepublik Deutschland, 55%ig in Wasser)
174 g gequollene, hochviskose Methylhydroxyethylcellulose (0,5%ig in Wasser)
336 g Kieselgur
336 g Titandioxid
0,95g Tetraethylammonium-perfluoroctansulfonat
40 g 0,5 M Phosphatpuffer pH 5,5
23 g Methanol
46 g 1-Hexanol
69 g Aceton
65 g Wasser
werden zu einer homogenen ersten Beschichtungsmasse verarbeitet und mit 0,18 mm Spalthöhe auf ein 0,20 mm dickes Polyester-Filtergewebe (2 F 777, Schweizer Seidengazefabrik Thal, Schweiz) beschichtet und getrocknet.

Auf den so erhaltenen, beschichteten Träger wird eine zweite Beschichtungsmasse bestehend aus
102 g Acrylsäureester-Copolymer-Dispersion (Acronal 14D der BASF, 55%ig in Wasser)
38 g gequollene, hochviskose Methylhydroxyethylcellulose (0,5%ig in Wasser)
3 g Natriumdodecylbenzolsulfonat
36 KU Glucoseoxidase
1050 KU Peroxidase
1,48 g 3,3′, 5,5′-Tetramethylbenzidin
0,53 g 1-Phenylsemicarbazid
28 g 1-Methoxy-2-propanol
40 g 1-Hexanol
38 g Wasser
die zu einer homogenen Masse verarbeitet wurden, mit 0,02 mm Spalthöhe aufgetragen und getrocknet.

Der so erhaltene Schichtverbund wird in einem Teststreifen gemäß Fig. 4 eingesetzt und ergibt eine gute Abstufung im Konzentrationsbereich von 20 - 250 mg Glucose/dl bei Verwendung von Vollblut.

### Beispiel 2

### Herstellung eines Testfelds für einen Testträger zum Triglycerid-Nachweis aus Blut

37 g Polyvinylpropionat-Dispersion (50%ig in Wasser)
29 g gequollene, hochviskose Methylhydroxyethylcellulose (0,5 %ig in Wasser)
56 g Kieselgur
56 g Titandioxid
3,2g Natriumdodecylbenzolsulfonat
40 g Phosphatpuffer 0,5 M, pH 5,5
3,8g Methanol
7,7g 1-Hexanol
11,5 g Aceton
22 g Wasser
werden zu einer homogenen ersten Beschichtungsmasse verarbeitet und mit 0,15 mm Spalthöhe auf ein 0,09 mm dickes Reinseidengewebe (Typ 541 der Spinnhütte Seidentechnik, Celle, Bundesrepublik Deutschland) beschichtet und getrocknet.

Auf den so erhaltenen Träger wird eine zweite Beschichtungsmasse, bestehend aus
20 g Polyvinylpropionat-Dispersion (50%ig in Wasser)
0,28 g Natriumalginat
70 g 0,2 M Phosphatpuffer pH 7,5
0,58 g Adenosin-5′-triphosphat, di-Natriumsalz
0,59 g Magnesiumsulfat-7-hydrat
1,0 g Dioctylnatriumsulfosuccinat
0,45 g 3,3′, 5,5′-Tetramethylbenzidin
15 mg 1-(4-Methylphenyl)-semicarbazid
16,4 g 1-Hexanol
30 g Aceton
2,0 g Triton® X-100
27 KU Cholesterinesterase
8,0 KU Glycerinphosphatoxidase
27 KU Glycerokinase
73 KU Peroxidase
50 g Wasser,
die zu einer homogenen Masse verarbeitet wurden, deren pH auf 7,5 eingestellt wurde, mit 0,01 mm Spalthöhe aufgetragen und getrocknet.

Die so erhaltenen Testfelder ergeben nach Auftüpfeln von Blut auf die Gewebeseite eine gute Abstufung im Konzentrationsbereich 100 - 300 mg Triglyceride/dl.

### Beispiel 3

### Herstellung eines Testfelds für einen Testträger zum Glucose-Nachweis aus Blut

Zu 46,3 g einer 20 Gew.%igen Lösung von Polyvinylacetat (Mowilith 70 der Fa. Höchst AG) in Aceton/1-Hexanol/Methanol (3:2:1, v/v) wird eine Lösung von 1,30 g Dioctylnatriumsulfosuccinat in 29,4 g Aceton gegeben. In dieser Mischung werden 28 g Kieselgur und 28 g Titandioxid dispergiert. Mit dieser homogenen ersten Beschichtungsmasse wird ein 0,20 mm dickes Polyester-Filtergewebe (2 F 777, Schweizer Seidengazefabrik, Thal, Schweiz) mit einer Spalthöhe von 0,2 mm beschichtet und getrocknet.

Auf den so erhaltenen beschichteten Träger wird eine zweite Beschichtungsmasse bestehend aus
64 g einer 20 Gew.-%igen Lösung von Polyvinylacetat (Mowilith 70) in Aceton/1-Hexanol/methanol (3:2:1, v/v)
1,3 g Dioctylnatriumsulfosuccinat
36 g Aceton
264 mg 1-Phenylsemicarbazid
740 mg 3,3′, 5,5′-Tetramethylbenzidin
13,8 g 1-methoxy-2-propanol,
die zu einer klaren zähflüssigen Lösung verarbeitet wurden, mit einer Spalthöhe von 0,04 mm aufgetragen und getrocknet.
Auf das so erhaltene Testfeld können die Reagenzien a) durch eine weitere Beschichtung, b) durch Sprühen aufgetragen werden:
a) Die Beschichtungsmasse für die Beschichtung besteht aus
   20 g gequollener, hochviskoser Methylhydroxyethylcellulose (0,5 %ig in Wasser)
   36 kU Glucoseoxidase
   1050 kU Peroxidase
   und wird mit einer Spalthöhe von 0,02 mm aufgetragen und getrocknet.
b) Die Sprühlösung besteht aus
   72 kU Glucoseoxidase
   2,1 MU Peroxidase
   40 ml Wasser
   und wird mit einem Verbrauch von 20-30 ml/m² augesprüht und getrocknet.

Die so erhaltenen Testfelder ergeben nach Auftüpfeln von Blut auf die Gewebeseite eine gute Abstufung im Konzentrationsbereich von 150-600 mg Glucose/dl.

## Patentansprüche

1. Testträger (1) zur Bestimmung eines Analyten aus Vollblut mit Hilfe von in dem Testträger (1) enthaltenen Reagenzien, mit einer Erythrozytenabtrenneinrichtung, die eine Blutaufgabeseite und eine Auswerteseite aufweist, wobei auf der Blutaufgabeseite Vollblut zugeführt wird und die Auswerteseite einer Nachweiseinrichtung zugewandt ist, in der infolge der Reaktion der Reagenzien mit dem Analyten eine optisch nachweisbare Veränderung stattfindet, **dadadurch gekennzeichnet**, daß die Erythrozytenabtrenneinrichtung einen Schichtverbund (3) einschließt, der eine erste Zone (5), welche einen polymeren Filmbildner, Kieselguhr und ein Pigment enthält und eine darauf unter Bildung eines Übergangsbereichs (7) durch Flüssigbeschichten geformte, einen polymeren Filmbildner enthaltende zweite Zone (6) aufweist, wobei die erste Zone (5) der Blutaufgabeseite (8) und die zweite Zone (6) der Auswerteseite (9) der Erythrozytenabtrenneinrichtung zugewandt ist.

2. Testträger nach Anspruch 1, **dadurch gekennzeichnet**, daß der Schichtverbund auf einer porösen Tragschicht (4) befestigt ist, wobei die Tragschicht der Blutaufgabeseite (8) zugewandt ist.

3. Testträger nach Anspruch 1, **dadurch gekennzeichnet**, daß mindestens einer der polymeren Filmbildner der ersten Zone (5) und der zweiten Zone (6) ein Dispersionsfilmbildner ist.

4. Testträger nach Anspruch 1, **dadurch gekennzeichnet**, daß die Kieselgur einen mittleren Teilchendurchmesser von 5-15 µm hat.

5. Testträger nach Anspruch 1, **dadurch gekennzeichnet**, daß das Pigment einen mittleren Teilchendurchmesser von 0,2 - 0,7 µm hat.

6. Testträger nach Anspruch 1, **dadurch gekennzeichnet**, daß die Kieselgur und das Pigment in der ersten Zone (5) in einem Gewichtsverhältnis von 1:0,5 bis 1:2 zueinander stehen.

7. Testträger nach Anspruch 1, **dadurch gekennzeichnet**, daß die Kieselgur und der polymere Filmbildner in der ersten Zone (5) in einem Gewichtsverhältnis von 1:0,2 bis 1:0,9 zueinander stehen.

8. Testträger nach Anspruch 1, **dadurch gekennzeichnet**, daß der Schichtverbund (3) eine Stärke von maximal 0,6 mm, bevorzugt maximal 0,2 mm, hat.

9. Verfahren zur Herstellung eines Reagenzien enthaltenden Testträgers nach Anspruch 1 wobei **dadurch gekennzeichnet**, daß man eine erste fließfähige Beschichtungsmasse herstellt, welche eine Lösung oder Dispersion eines polymeren Filmbildners in einer geeigneten Trägerflüssigkeit, gemischt mit Kieselgur, einem Pigment und Hilfsstoffen enthält, eine zweite fließfähige Beschichtungsmasse herstellt, welche eine Lösung oder Dispersion eines polymeren Filmbildners enthält und zur Herstellung des Schichtverbundes zunächst die erste Beschichtungsmasse auf einer Unterlage in einer dünnen Schicht ausformt und trocknet und dann auf dieser Schicht die zweite Beschichtungsmasse zu einer dünnen Schicht ausformt und trocknet.

10. Verfahren nach Anspruch 9, **dadurch gekennzeichnet**, daß die Unterlage, auf der man die erste Beschichtungsmasse ausformt, eine poröse Tragschicht ist.

11. Verfahren nach Anspruch 9, **dadurch gekennzeichnet**, daß die zweite Beschichtungsmasse eine ein optisch nachweisbares Signal produzierende Komponente enthält.

12. Verfahren nach Anspruch 9, **dadurch gekennzeichnet**, daß die erste Beschichtungsmasse beim Aufbringen auf die Unterlage eine Viskosität von 300 bis 3000 mPas (Millipascalsekunden) bei einem Schergefälle von 492 s⁻¹ nach DIN 53019 hat.

13. Verfahren nach Anspruch 9, **dadurch gekennzeichnet**, daß die zweite Beschichtungsmasse beim Aufbringen auf die erste Schicht eine Viskosität von 100 bis 1000 mPas (Millipascalsekunden) bei einem Schergefälle von 492 s⁻¹ nach DIN 53019 hat.

14. Verfahren nach Anspruch 9, **dadurch gekennzeichnet**, daß die zweite Schicht mit einem Flächengewicht von maximal 100 g polymerem Filmbildner pro m² ausgeformt wird.

## Claims

1. Test carrier (1) for the determination of an analyte from whole blood with the help of reagents contained in the test carrier (1), with an erythrocyte separation means which has a blood application side and an evaluation side, wherein whole blood is supplied to the blood application side and the evaluation side faces a detection means in which, as a result of the reaction of the reagents with the analyte, an optically detectable change takes place, characterised in that the erythrocyte separation means includes a laminated composite (3) which has a first zone (5), which contains a polymeric film former, kieselguhr and a pigment, and thereupon, with formation of a transition region (7), has a second zone (6) containing a polymeric film-former, formed by liquid coating, the first zone (5) facing the blood application side (8) and the second zone (6) facing the evaluation side (9) of the erythrocyte separation device.

2. Test carrier according to claim 1, characterised in that the laminated composite is fixed to a porous carrier layer (4), the carrier layer facing the blood application side (8).

3. Test carrier according to claim 1, characterised in that at least one of the polymeric film formers of the first zone (5) and of the second zone (6) is a dispersion film former.

4. Test carrier according to claim 1, characterised in that the kieselguhr has an average particle diameter of 5 - 15 µm.

5. Test carrier according to claim 1, characterised in that the pigment has an average particle diameter of 0.2 - 0.7 µm.

6. Test carrier according to claim 1, characterised in that the kieselguhr and the pigment in the first zone (5) stand in a weight ratio to one another of 1:0.5 to 1:2.

7. Test carrier according to claim 1, characterised in that the kieselguhr and the polymeric film former in the first zone (5) stand in a weight ratio to one another of 1:0.2 to 1:0.9.

8. Test carrier according to claim 1, characterised in that the laminated composite (3) has a thickness of maximum 0.6 mm, preferably maximum 0.2 mm.

9. Process for the production of a reagent-containing test carrier according to claim 1, characterised in that one produces a first flowable coating mass which contains a solution or dispersion of a polymeric film former in a suitable carrier liquid mixed with kieselguhr, a pigment and adjuvant materials, produces a second flowable coating mass which contains a solution or dispersion of a polymeric film former, and, for the production of the laminated composite, first forms the first coating mass on a substrate in a thin layer and dries and then, on this layer, forms the second coating mass to give a thin layer and dries.

10. Process according to claim 9, characterised in that the substrate on which the first coating mass is formed is a porous support layer.

11. Process according to claim 9, characterised in that the second coating mass contains a component producing an optically detectable signal.

12. Process according to claim 9, characterised in that the first coating mass, when applied to the substrate, has a viscosity of 300 to 3000 mPas (millipascal seconds) with a shear gradient of 492 s⁻¹ according to DIN 53019.

13. Process according to claim 9, characterised in that the second coating mass, when applied to the first layer, has a viscosity of 100 to 1000 mPas (millipascal seconds) with a shear gradient of 492 s⁻¹ according to DIN 53019.

14. Process according to claim 9, characterised in that the second layer is formed with a weight per unit surface area of maximum 100 g of polymeric film former per m².

## Revendications

1. Support de test (1) pour la détermination d'un analyte de sang complet, à l'aide de réactifs contenus dans le support de test (1), avec un dispositif de séparation des érythrocytes, qui présente une face d'application du sang et une face de détermination du sang, du sang complet étant déposé sur la face d'application du sang et la face de détermination du sang étant tournée vers un dispositif de détermination dans lequel, à la suite de la réaction des réactifs avec l'analyte, il se produit une modification détectable optiquement, caractérisé en ce que le dispositif de séparation des érythrocytes comprend une couche composite (3) qui présente une première zone (5) contenant un formateur de film polymère, de la terre d'infusoire et un pigment, et une seconde zone (6) formée par enduction d'un liquide, contenant un formateur de film polymère, disposée sur la première en formant une zone de transition (7), la première zone (5) étant la face d'application du sang (8) et la seconde zone (6) étant tournée vers la face de détermination (9) du dispositif de séparation des érythrocytes.

2. Support de test selon la revendication 1, caractérisé en ce que la couche composite est fixée sur une couche de support (4) poreuse, la couche de support étant tournée vers la face d'application du sang (8).

3. Support de test selon la revendication 1, caractérisé en ce qu'au moins l'un des formateurs de film polymère de la première zone (5) et de la seconde zone (6) est un formateur de film en dispersion.

4. Support de test selon la revendication 1, caractérisé en ce que la terre d'infusoire présente une taille moyenne des particules de 5-15 µm.

5. Support de test selon la revendication 1, caractérisé en ce que le pigment présente une taille moyenne des particules de 0,2-0,7 µm.

6. Support de test selon la revendication 1, caractérisé en ce que la terre d'infusoire et le pigment sont, dans la première zone (5), dans un rapport pondéral entre eux de 1 : 0,5 à 1 : 2.

7. Support de test selon la revendication 1, caractérisé en ce que la terre d'infusoire et le formateur de film polymère sont, dans la première zone (5), dans un rapport pondéral entre eux de 1 : 0,2 à 1 : 0,9.

8. Support de test selon la revendication 1, caractérisé en ce que la couche composite (3) présente une épaisseur maximale de 0,6 mm, de préférence de 0,2 mm.

9. Procédé de préparation d'un support de test contenant des réactifs selon la revendication 1, caractérisé en ce que l'on prépare une première masse d'enduction fluide, qui contient une solution ou dispersion appropriée d'un formateur de film polymère dans un véhicule liquide approprié, mélangé à de la terre d'infusoire, un pigment et des adjuvants, on prépare une seconde masse d'enduction fluide, qui contient une solution ou dispersion d'un formateur de film polymère, et pour la préparation de la couche composite, on dépose d'abord la première masse d'enduction sur un support en une couche mince et la sèche, et ensuite, on dépose sur cette couche la seconde masse d'enduction en une couche mince, et la sèche.

10. Procédé selon la revendication 9, caractérisé en ce que le support sur lequel on dépose la première masse d'enduction est une couche de support poreuse.

11. Procédé selon la revendication 9, caractérisé en ce que la seconde masse d'enduction contient un composant produisant un signal optiquement détectable.

12. Procédé selon la revendication 9, caractérisé en ce que la première masse d'enduction présente, lors de l'application sur le support, une viscosité de 300 à 3000 mPa·s (millipascalsecondes), pour une chute de 492 s⁻¹, selon DIN 53019.

13. Procédé selon la revendication 9, caractérisé en ce que la seconde masse d'enduction présente, lors de l'application sur la première couche, une viscosité de 100 à 1000 mPa·s (millipascalsecondes), pour une chute de 492 s⁻¹, selon DIN 53019.

14. Procédé selon la revendication 9, caractérisé en ce que la seconde couche est formée avec un poids spécifique de 100 g au plus par m² de formateur de film polymère.
